# EUROPEAN PATENT APPLICATION

(11) **EP 3 009 282 A1**
(43) Date of publication of application: **20.04.2016**
(21) Application number: 15187160.5
(22) Date of filing: 28.09.2015
(51) Int. Cl.: B60H 1/00, B60N 2/00, B60R 21/015

(54) **OCCUPANT SAFETY SYSTEM WITH CO2 DETECTION**

(30) Priority: 14.10.2014 US 201414513943
(71) Applicant: Delphi Technologies, Inc., Troy, MI 48007 (US)
(72) Inventor: GRIFFIN, Dennis P., Noblesville, INDIANA 46062 (US)
(74) Representative: Delphi France SAS

(57) **Abstract**

An occupant (20) safety system (10) for a vehicle (12) includes a carbon-dioxide (CO2) sensor and a controller (22). The CO2 sensor is configured to determine a CO2 concentration in a passenger cabin (18) of the vehicle (12) while the vehicle (12) is not operating, i.e. parked. The controller (22) is configured to determine that the passenger cabin (18) is occupied by an occupant (20) based on the CO2 concentration in a passenger cabin (18) while the vehicle (12) is not operating. If the CO2 concentration is a concern while the vehicle (12) is not operating, the system (10) may respond by activating a notification means such as a vehicle (12) horn or alarm, or by ventilating the passenger cabin (18) via opening the windows or operating a blower of a HVAC system (10) of the vehicle (12).

## Description

### TECHNICAL FIELD

This disclosure generally relates to an occupant safety system for a vehicle, and more particularly relates to equipping the vehicle with a CO2 sensor to determine a CO2 concentration in a passenger cabin while the vehicle is not operating (i.e. parked).

### BACKGROUND OF THE INVENTION

There is a desire to protect occupants of vehicle while the vehicle is not operating, e.g. parked and the engine not running. Occupants such as infants inadvertently left in the vehicle, elderly persons waiting for someone to return from shopping, or abled bodied persons sleeping in the vehicle are examples of occupants present in a vehicle while the vehicle is not operating.

### SUMMARY OF THE INVENTION

In accordance with one embodiment, an occupant safety system for a vehicle is provided. The system includes a carbon-dioxide (CO2) sensor and a controller. The CO2 sensor is configured to determine a CO2 concentration in a passenger cabin of the vehicle while the vehicle is not operating. The controller is configured to determine that the passenger cabin is occupied by an occupant based on the CO2 concentration in a passenger cabin while the vehicle is not operating.

In another embodiment, a method of operating an occupant safety system of a vehicle is provided. The method includes the step of providing a CO2 sensor configured to determine a CO2 concentration in a passenger cabin of the vehicle while the vehicle is not operating. The method also includes the step of determining that the passenger cabin is occupied by an occupant based on the CO2 concentration in a passenger cabin while the vehicle is not operating.

Further features and advantages will appear more clearly on a reading of the following detailed description of the preferred embodiment, which is given by way of non-limiting example only and with reference to the accompanying drawings.

### BRIEF DESCRIPTION OF THE DRAWINGS

The present invention will now be described, by way of example with reference to the accompanying drawings, in which:
Fig. 1 is a diagram of a vehicle equipped with a system to detect CO2 concentrations in a vehicle cabin while the vehicle is not operating in accordance with one embodiment; and
Fig. 2 is a flow chart of a method for operating the system of Fig. 1 in accordance with one embodiment.

### DETAILED DESCRIPTION OF THE PREFERRED EMBODIMENT

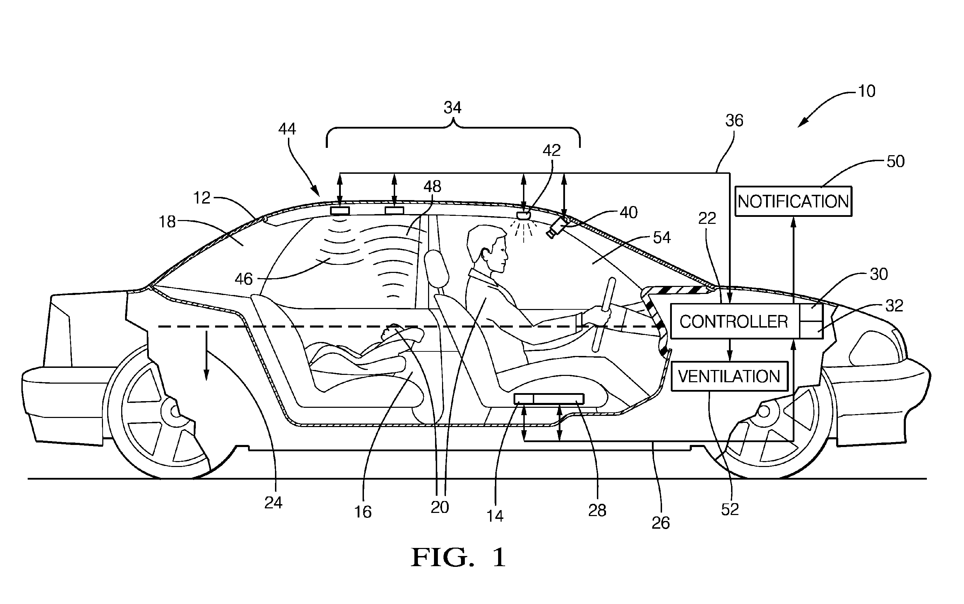

Fig. 1 illustrates a non-limiting example of an occupant safety system, hereafter the system 10, suitable for use in a vehicle 12. The system 10 includes a carbon dioxide (CO2) sensor, hereafter the CO2 sensor 14, configured to determine a CO2 concentration 16 in a passenger cabin 18 of the vehicle 12. Suitable CO2 sensors that use a version of spectroscopy to isolate and measure the CO2 gas concentration are commercially available. The system 10 takes advantage of the reliability of this type of CO2 sensing to provide a safety system that could be installed in the vehicle as a standalone system, or integrated into one of the vehicle's existing microcomputer based devices.

The system 10 described herein is particularly intended to be used while the vehicle is not operating. As used herein, the term 'not operating' refers to a condition when the vehicle is parked, and the engine (not shown) of the vehicle is not running. This 'not operating' condition is the same or similar to the condition of the vehicle 12 when, for example, the vehicle is not in use while being stored in a garage over-night, or when the vehicle is parked in a parking lot while the owner/operator of the vehicle is working or shopping.

The system 10 described herein measures the CO2 concentration 16 to provide an indication of the quality of air of the passenger cabin 18. Since CO2 is a byproduct of respiration, an enclosed space like the passenger cabin 18 may exhibit increasing levels of CO2 if sufficient fresh air is not provided to the passenger cabin 18. Testing suggests that if the CO2 concentration 16 is greater than about one-thousand parts-per-million (1000ppm), an occupant 20 of the passenger cabin 18 may experience noticeable levels of drowsiness and/or discomfort. For most people an increased respiration rate occurs at 2000ppm. At 20000ppm, respiration rate increases by 50%. At 30000ppm, respiration rate increases by 100%, and a maximum recommended Permissible Exposure Limit (PEL) is 15 minutes. It is recognized that an individual's age, health and respiratory condition can dramatically affect these levels with regard to individual safe levels of CO2.

It has been observed that when an adult breathes in a typical automobile passenger cabin with the windows closed and the vehicle not operating, the CO2 concentration 16 increases from around 300ppm (typical fresh air concentration is 250ppm to 400ppm) to more than 1500ppm within 15 minutes. Without sufficient fresh air, occupants such as young children, the elderly, and/or pets that are unable to operate the vehicle to provide adequate fresh air may be exposed to undesirable CO2 concentrations. In contrast, an unoccupied passenger cabin exhibits very little variation and nearly constant levels of CO2. Over time with no occupants in a vehicle, the CO2 levels will gradually fall to match the ambient levels outside of the vehicle due to small air leaks in the seals of the passenger cabin 18.

The system 10 includes a controller 22 configured to determine that the passenger cabin 18 is occupied by an occupant based on the CO2 concentration in a passenger cabin while the vehicle is not operating. As used herein, the term occupant is generally directed to living animals such as, but is not limited to, humans, dogs, cats, and other animals that may occupy a passenger cabin while the vehicle 12 is parked. While the example given in Fig. 1 suggests that the system is used in an automobile, the teachings presented herein may also be used to protect livestock while being transported.

The controller 22 may include a processor (not shown) such as a microprocessor or other control circuitry such as analog and/or digital control circuitry including an application specific integrated circuit (ASIC) for processing data as should be evident to those in the art. The controller 22 may include memory, including non-volatile memory, such as electrically erasable programmable read-only memory (EEPROM) for storing one or more routines, thresholds and captured data. The one or more routines may be executed by the processor to perform steps for determining if an occupant is present in the passenger cabin 18 based on signals received by the controller 22 as described herein.

Because CO2 has a higher molecular density (@44) than the major components of typical atmosphere, those being N2 (@28) and 02 (@32), there may be instances when CO2 settles to the lower areas of the passenger cabin 18. As such, it may be advantageous if the CO2 sensor 14 is located in a lower third 24, volume-wise, of the passenger cabin 18. Since the configuration of most passenger cabins is such that the lower regions are occupied by seating and the like, while the upper regions are more open, the line indicating the boundary of the lower third 24 will likely be higher than one third of the distance from the floor to the ceiling of the passenger cabin, possibly about mid-way for example.

It may also be advantageous if the CO2 sensor 14 is part of a seat-occupancy detection device 26 that detects the presence of the occupant 20 based on, for example, weight or capacitive coupling, as will be recognized by those in the art. Integration of the CO2 sensor 14 with the seat-occupancy detection device 26 would avoid some duplication of electronics such as power supply filtering/regulation. The CO2 sensor may be electrically coupled to the controller 22 by way of shared data bus 28. Alternatively, a dedicated wire between the CO2 sensor and the controller 22 may be used to communicate the CO2 concentration 16, or the communication may be by way of a wireless means.

The controller 22 may be configured to determine that the passenger cabin 18 is occupied if the CO2 concentration 16 is greater than a concentration threshold 30 stored in the controller 22. A suitable value for the concentration threshold 30 is 1000ppm; however it is believed that lower values may be used without a significant risk of false alarms, a value of 700ppm for example. Alternatively, instead of waiting for the CO2 concentration 16 to exceed the concentration threshold 30, the controller 22, may be configured to determine that the passenger cabin 18 is occupied if the CO2 concentration 16 increases at a rate greater than a rate threshold 32. A suitable value for the rate threshold 32 is thirty parts per million per minute (30ppm/min). However it is recognized that the system 10 may be calibrated with a different value if the passenger cabin 18 is substantially larger or smaller than the passenger cabin depicted herein.

Other techniques to determine the presence of an occupant have been considered, but these other techniques do not directly protect the occupant 20 from excessive CO2 concentrations, and have some fundamental problems with actually determining that the occupant 20 is indeed present. For example, techniques that use cameras or ultrasonic transducers to determine that an occupant is present based on detected movement by the occupant 20 may fail if the occupant is sleeping and not moving. Systems that rely on detecting body heat radiated by the occupant 20 may fail if the occupant 20 is covered with a blanket. Nevertheless, the probability of the system 10 detecting that the passenger cabin 18 is occupied may be increased by combining these other techniques with direct detection of CO2 concentration in a passenger cabin 18. That is, the system 10 may advantageously include a cabin-occupancy detection device 34 configured to output a detection signal 36 indicative of an occupant presence in the passenger cabin 18. Accordingly, the controller 22 may be further configured to determine that the passenger cabin 18 is occupied based on both the detection signal 36 and the CO2 concentration 16 in a passenger cabin 18.

The cabin-occupancy detection device 34 may include a camera 40 configured to view areas of the passenger cabin 18 were the occupant 20 may reside. The detection signal 36 may be based on one of visible light and near infrared light present in the passenger cabin 18, or alternatively it may be based on both visible light and near infrared light present in the passenger cabin 18. The controller 22 may then be configured to detect the presence of the occupant 20 by, for example, motion flow analysis of images from the camera 40, or by other types of image analysis as will be recognized by those in the art. The cabin-occupancy detection device 34 may also include an illuminator 42 configured to emit light such as near infrared light so the camera 40 can capture images if the ambient illumination level of the passenger cabin 18 is low.

Alternatively, the camera 40 may be configured to detect far-infrared light, i.e. thermal energy radiated by the occupant 20 residing in the vehicle cabin. Detecting thermal energy, i.e. detecting the temperature of an object, may be advantageous as doing so is not dependent on illumination from the illuminator 42. However, the resolution of such a device is relatively low when compared to a visible light or near infrared type camera with similar cost. In another embodiment, the cabin-occupancy detection device 34 may include an ultra-sonic transducer 44 configured to emit an ultrasonic signal 46 and detect ultrasonic sound waves 48. Accordingly, the detection signal 36 may be indicative of the ultrasonic sound waves 48 reflected in the passenger cabin 18. It is recognized that the camera 40, with or without the illuminator 42, could be used in combination with the ultrasonic transducer 44 to further increase the probability that the presence of the occupant 20 is detected when compared to using the CO2 sensor 14 alone.

In response to detecting or determining that the CO2 concentration 16 is greater than the concentration threshold 30, and/or the rate at which the CO2 concentration 16 is increasing is greater than the rate threshold 32, the system 10 may include a notification means 50 operable by the controller 22 to indicate that the passenger cabin 18 is occupied and that the CO2 concentration 16 is a concern. The notification means 50 may indicate to persons outside of the passenger cabin 18 (i.e. outside of the vehicle 12) that the passenger cabin 18 is occupied and needs attention by any one or combination of notification techniques such as, but not limited to, flashing a headlight or other exterior lights of the vehicle, sounding or activating the vehicle's horn, and transmitting a warning to a designated phone number via a cellular phone network. If the CO2 level is dangerously high and the presence of an occupant is highly likely, the controller 22 may be configured to unlock the doors of the vehicle 12 so persons outside of the vehicle 12 can assist the occupant 20.

Alternatively, or in addition to activating the notification means 50, the system 10 may include a ventilation means 52 operable by the controller 22 to provide fresh air to the passenger cabin 18 while the vehicle 12 is not operating. The ventilation means 52 may include a window 54 equipped with an actuator capable of opening (e.g. rolling down) the window 54 while the vehicle is not operating to allow fresh air to enter the passenger cabin 18. By way of further example and not limitation, the ventilation means 52 may include a fan or blower such as the blower of the vehicle's heating, ventilation, and air-conditioning (HVAC) system operable by the controller 22 to draw fresh air into the passenger cabin 18 while the vehicle 12 is not operating.

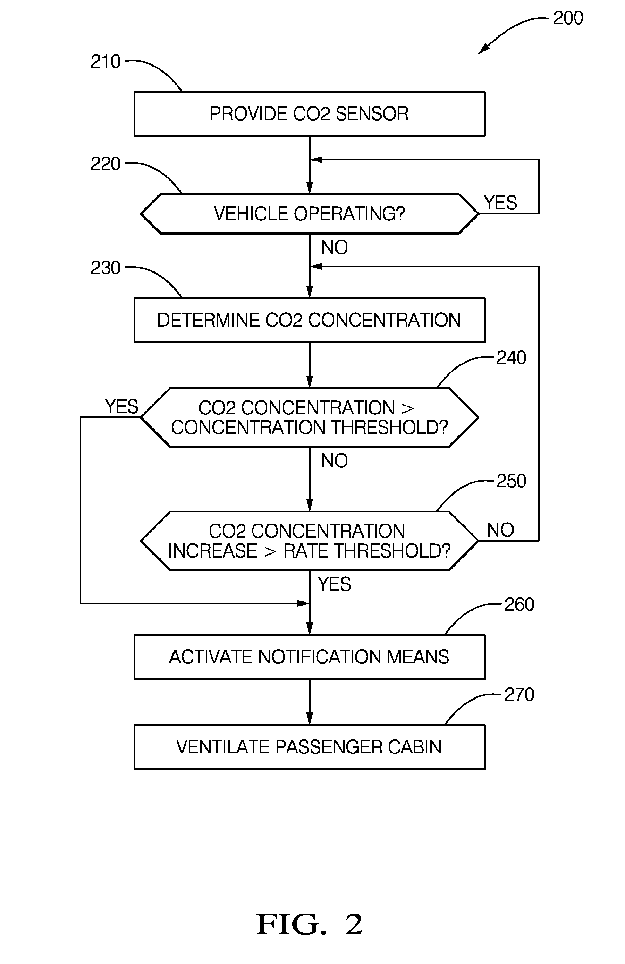

Fig. 2 illustrates a non-limiting example of method 200 of operating an occupant safety system (the system 10) in the vehicle 12.

Step 210, PROVIDE CO2 SENSOR, may include providing or installing the CO2 sensor 14 in the vehicle 12 either as a stand-alone sensor, or integrated into the seat occupancy detection device 26, or integrated in to some preexisting vehicle control unit. In general, the CO2 sensor is configured to determine the CO2 concentration 16 in a passenger cabin 18 ofthe vehicle 12 while the vehicle is not operating.

Step 220, VEHICLE OPERATING?, may include detecting a voltage from an engine control unit or a voltage affected by the position of the ignition key to determine if the engine is running. Alternatively, the system 10 may include a vibration sensor (not shown) so that the state of vehicle operation could be determined independent of other vehicle systems.

Step 230, DETERMINE CO2 CONCENTRATION, may include determining that the passenger cabin 18 is occupied by an occupant 20 based on the CO2 concentration in a passenger cabin 18 while the vehicle is not operating. The CO2 concentration 16 indicated by the CO2 sensor 14 may be filtered or otherwise processed by the controller 22 to improve signal-to-noise ratio or calculate a rate of change of the CO2 concentration 16.

Steps 240 and 250 show different ways to determine if the CO2 concentration 16 is a concern. While the method 200 shows both steps, it is recognized that only one or only the other may be performed and still detect excessive CO2 concentrations.

Step 240, CO2 CONCENTRATION > CONCENTRATION THRESHOLD?, may include determining if the CO2 concentration 16 is greater than a concentration threshold 30. The concentration threshold 30 may be preprogrammed into the controller 22, and/or may be adjusted over time to compensate for calibration drift of the CO2 sensor 14 and/or normal ambient concentrations of CO2.

Step 250, CO2 CONCENTRATION INCREASE > RATE THRESHOLD?, may include determining if the CO2 concentration 16 is increasing at a rate greater than a rate threshold 32. To determine the rate of CO2 concentration increase, the controller 22 may sample the CO2 concentration 16 reported by the CO2 sensor 14 over a period of time such as a few minutes. The rate threshold 32 may be preprogrammed into the controller 22, and/or may be adjusted over time to compensate for calibration drift of the CO2 sensor 14 and/or normal fluctuations in ambient concentrations of CO2.

Steps 260 and 270 show different ways to respond if the CO2 concentration 16 is a concern. While the method 200 shows both steps, it is recognized that only one or only the other may be performed and still respond to excessive CO2 concentrations.

Step 260, ACTIVATE NOTIFICATION MEANS, may include activating a notification means 50 to indicate that the passenger cabin 18 is occupied. The notification means may be, for example, illuminating vehicle exterior lights, sounding the horn or other audible alarm, or texting a message to a phone number on a cellular phone network.

Step 270, VENTILATE PASSENGER CABIN, may include ventilating the passenger cabin while the vehicle is not operating by the controller 22 operating a power window of the vehicle 12 to an open state, or operating the blower of the vehicle HVAC system to blow fresh air into the passenger cabin 18.

Accordingly, an occupant safety system (the system 10) for a vehicle 12, and a method 200 of operating the system 10 of a vehicle 12 is provided. While it is recognized that the CO2 sensor 14 may also be useful while the vehicle is operating (e.g. while being driven), the solution to the problem of protecting the occupant 20 from excessive CO2 concentrations while the vehicle 12 is not operating is the focus of the system 10 and method 200 described herein.

While this invention has been described in terms of the preferred embodiments thereof, it is not intended to be so limited, but rather only to the extent set forth in the claims that follow.

## Claims

1. An occupant safety system (10) for a vehicle (12), said system (10) comprising:
a CO2 sensor (14) configured to determine a CO2 concentration (16) in a passenger cabin (18) of the vehicle (12) while the vehicle (12) is not operating; and
a controller (22) configured to determine that the passenger cabin (18) is occupied by an occupant (20) based on the CO2 concentration (16) in a passenger cabin (18) while the vehicle (12) is not operating.

2. The system (10) in accordance with claim 1, wherein the CO2 sensor (14) is located in a lower third (24), volume-wise, of the passenger cabin (18).

3. The system (10) in accordance with claim 1 or 2, wherein the CO2 sensor (14) is part of a seat-occupancy detection device (26).

4. The system (10) in accordance with any one of claims 1 to 3, wherein the controller (22) determines that the passenger cabin (18) is occupied if the CO2 concentration (16) is greater than a concentration threshold (30).

5. The system (10) in accordance with any one of claims 1 to 4, wherein the controller (22) determines that the passenger cabin (18) is occupied if the CO2 concentration (16) is increasing at a rate greater than a rate threshold (32).

6. The system (10) in accordance with any one of claims 1 to 5, wherein the system (10) includes
a cabin-occupancy detection device (34) configured to output a detection signal (36) indicative of an occupant (20) presence in the vehicle (12) cabin, wherein
the controller (22) is further configured to determine that the passenger cabin (18) is occupied based on the detection signal (36) and the CO2 concentration (16) in a passenger cabin (18).

7. The system (10) in accordance with claim 6, wherein the detection signal (36) is based on at least one of visible light and near infrared light present in the vehicle (12) cabin.

8. The system (10) in accordance with claim 6, the detection signal (36) is based on thermal energy radiated in the vehicle (12) cabin.

9. The system (10) in accordance with claim 6, the detection signal (36) is indicative of ultrasonic sound waves (48) reflected in the vehicle (12) cabin.

10. The system (10) in accordance with any one of claims 1 to 9, wherein the system (10) includes a notification means operable by the controller (22) to indicate that the passenger cabin (18) is occupied.

11. A method (200) of operating an occupant (20) safety system (10) of a vehicle (12), said method (200) comprising:
providing (210) a CO2 sensor (14) configured to determine a CO2 concentration (16) in a passenger cabin (18) of the vehicle (12) while the vehicle (12) is not operating; and
determining (220, 230, 240, 250) that the passenger cabin (18) is occupied by an occupant (20) based on the CO2 concentration (16) in a passenger cabin (18) while the vehicle (12) is not operating (220).

12. The method (200) in accordance with claim 11, wherein determining that the passenger cabin (18) is occupied includes determining (240) if the CO2 concentration (16) is greater than a concentration threshold (30).

13. The method (200) in accordance with claim 11 or 12, wherein determining that the passenger cabin (18) is occupied includes determining (250) if the CO2 concentration (16) is increasing at a rate greater than a rate threshold (32).

14. The method (200) in accordance with any one of claims 11 to 13, wherein the method (200) includes activating (260) a notification means to indicate that the passenger cabin (18) is occupied.

15. The method (200) in accordance with any one of claims 11 to 14, wherein the method (200) includes ventilating (270) the passenger cabin (18) while the vehicle (12) is not operating.
